# EUROPEAN PATENT APPLICATION

(11) **EP 4 636 068 A1**
(43) Date of publication of application: **22.10.2025**
(21) Application number: 24171329.6
(22) Date of filing: 19.04.2024
(51) Int. Cl.: C12M 1/00, C12M 1/02, C12M 1/34

(54) **SINGLE-USE CLOSED BIOREACTOR FOR CULTURING, FERMENTING OR PROCESSING A BIOMASS**

(71) Applicant: Sartorius Stedim Biotech GmbH, 37079 Göttingen (DE)
(72) Inventor: Schubert, Jan-Eike, 37079 Göttingen (DE); Leupold, Marco, 34302 Guxhagen (DE); Maier, Friedrich, 37079 Göttingen (DE); Saral, Mertcan, 37079 Göttingen (DE); Purshouse, Martin, Gloucestershire, GL10 3UT (GB)
(74) Representative: Gottschald Patentanwälte Partnerschaft mbB

(57) **Abstract**

The invention relates to a single-use closed bioreactor for culturing, fermenting or processing a biomass with a rigid housing (3) with an interior space (4) with an inner height, an inner width and a volume smaller 100 L, wherein the rigid housing (3) comprises a lid (5) with an upper surface (8) and a lower surface (9) and a body, wherein the body comprises a wall (6), a top and a closed bottom (7) opposite to the top, wherein the top of the body is irreversibly connected to the lid (5) such that the top is closed by the lid (5), wherein the lid (5) comprises a cooling structure (40) for cooling an exhaust gas of the bioreactor (1), wherein the cooling structure (40) comprises a cooling channel (41) with a channel ceiling (42) and a channel floor (43), wherein the cooling channel (41) has a channel inlet (44) at the lower surface (9) of the lid (5) within the interior space (4) of the housing and a channel outlet (45) at the upper surface (8) of the lid (5) outside the housing, wherein said cooling channel (41) has a horizontal section (46) which is at least partially oriented horizontally within the lid (5).

## Description

The present invention relates to a single-use closed bioreactor for culturing, fermenting or processing a biomass according to claim 1, to a bioreactor arrangement according to claim 13, to a use of a single-use bioreactor according to claim 14 and to a method of production of a single-use bioreactor according to claim 15.

A single-use (closed) bioreactor is generally understood to be an apparatus in which microorganisms or mammalian cells, sometimes in combination with viruses, are cultivated under given conditions and which is normally discarded after finishing the cultivation process and subsequent harvest. Such bioreactors typically comprise a housing, in which a liquid such as cell culture comprising a biological nutrient medium with substances intended for the cultivation on the one hand and cells such as mammalian cells on the other hand, are accommodated and cultivated under certain conditions. In order to be able to carry out the respective cultivation process, various parameters being critical for the metabolic activity of the cells have to be adjusted such as nutrient medium composition, pH, temperature or the like. The product is then usually further treated in a so-called downstream process to obtain a finally purified product.

A known single-use bioreactor according to EP 2 586 857 A1 is related to a rigid single-use closed bioreactor. This single-use closed bioreactor comprises a cooling channel. Another rigid single-use closed bioreactor is known from EP 2 141 224 A1 and comprises a bottom through port.

Besides the bioreactors comprising a rigid housing also flexible bag bioreactors exist. These types of bioreactor comprise a bag made of a flexible shape-conforming material which serves as enclosure for receiving a liquid such as a cell culture. However, due to their flexible and not self-supporting nature these types of bioreactor require a bioprocess container shell as support structure which prevents the bag from collapsing when being filled with the liquid. Furthermore, these flexible bags are differently manufactured, differently transported, differently used and have different challenges when producing stirrers and other components on the inside, which need to conform to the flexible nature of the bag.

A rigid structure of a rigid housing for example prevents elements located within the rigid housing such as the rigid support structure or the stirrer shaft from being damaged during transportation. Due to its rigid nature the rigid housing absorbs impact shocks without transmitting them to the elements located within the rigid housing as they are located safely in the interior space of the rigid housing. Rigid bioreactors provide for easier handling in many situations, however, also require more space and more complexity in some areas.

It is an object of the present invention to provide a more flexible rigid bioreactor that can be used for more use cases, possibly for scaling and may have different variants such that a user can learn a single design for several applications. In particular, a flexible rigid bioreactor with a size between 1 L and 10 L is needed.

The above-noted object is solved by the features of claim 1.

The main realization of the present invention is that a closed bioreactor may be flexibly designed with a cooling channel to cool an exhaust gas.

In detail, a single-use closed bioreactor for culturing, fermenting or processing a biomass with a rigid housing with an interior space with an inner height, an inner width and a volume smaller 100 L, wherein the rigid housing comprises a lid with an upper surface and a lower surface and a body, wherein the body comprises a wall, a top and a closed bottom opposite to the top, wherein the top of the body is irreversibly connected to the lid such that the top is closed by the lid, wherein the lid comprises a cooling structure for cooling an exhaust gas of the bioreactor, wherein the cooling structure comprises a cooling channel with a channel ceiling and a channel floor, wherein the cooling channel has a channel inlet at the lower surface of the lid within the interior space of the housing and a channel outlet at the upper surface of the lid outside the housing, wherein said cooling channel has a horizontal section which is at least partially oriented horizontally within the lid is proposed.

In one embodiment, the body comprises a bottom through port and/or the bioreactor comprises a stirrer shaft, wherein the stirrer shaft is arranged on the lid of the bioreactor and extends from the lid into the interior space of the rigid housing, wherein the stirrer shaft comprises a magnetic component, which is configured to interact with a magnetic drive located outside the body, and/or the bioreactor comprises a first rigid support structure extending from the lid into the interior space of the rigid housing, wherein the first rigid support structure comprises a, in particular rigid, first conduit, wherein the lid comprises a first inlet port connected to the first conduit via which a fluid can be exchanged with the interior space of the rigid housing through the first conduit.

In one embodiment the first rigid support structure comprises multiple first conduits each being connected to a first inlet port of the lid. As the first rigid support structure provides stability to the multiple first conduits, such an embodiment prevents the different multiple first conduits from oscillating in a flow of a stirred liquid contained by the rigid housing thereby preventing the multiple first conduits becoming entangled. By reducing this oscillation uncontrolled disturbances are minimized and less turbulences are added to the flow of the liquid compared to multiple conduits not being stabilized by a rigid support structure which positively reduces additional shear forces. Such an embodiment further allows the addition of different liquids to the bioreactor and/or the removal of liquid from the bioreactor by means of different conduits.

According to a preferred embodiment of the single-use closed bioreactor the multiple first conduits end at different inner heights. This allows an exchange of fluid at different inner heights, preferably allowing the removal of a liquid such as cell culture from the interior space at different inner heights. Such a configuration is especially advantageous in microcarrier cell cultures where adherent mammalian cells are cultivated on microcarriers which are distributed within a cell culture medium and for other types of aggregated cells, cells that flock together and other not homogeneously distributed cells. During a settling phase in which the microcarriers settle to the closed bottom of the bioreactor and after settling, sampling can be done at different phases (different heights) and conceivably even the supernatant can be removed from the rigid housing and be separated from the cells by means of a dedicated conduit having an end being located above the settled microcarriers while other ones of the first multiple conduits at other heights may be used for example for the addition of liquid such as fresh cultivation medium during and/or outside the settling phase.

According to a preferred embodiment of the single-use closed bioreactor wherein the bioreactor comprises a second rigid support structure. When both, the first and second rigid support structure are located within a similar radial distance to a rotational axis of the stirrer shaft, shear forces can advantageously be reduced. Preferably, the second rigid support structure comprises conduits for gas. In one embodiment a second rigid support structure comprising an aeration device with one or more group of holes each for its own type of gas is provided. Such an embodiment allows a fluid such as cell culture medium in the rigid housing to be supplied with one or more gases. This advantageously allows the supply of oxygen required for cell metabolism and carbon dioxide for pH adjustment.

In another embodiment the first and second rigid support structure are structurally connected to each other via the aeration device. This configuration enables a force to be transferred from one structure to the other one thereby positively effecting the translational and/or torsional rigidity of the first and second rigid support structure leading to a reduced oscillation of the first and/or second rigid support structure.

According to embodiments the rigid housing of the bioreactor comprises an exhaust which allows a gas, preferably an aeration gas, to be discharged from the interior space of the rigid housing thereby preventing a pressure built-up and allowing a qualitative and/or quantitative analysis of the exhaust gas for specific components when being connected to an appropriate exhaust gas analysis device. The proposed exhaust may be connected to a filter element, preferably via a Y-connector piece comprising a first Y-connector port and a second Y-connector port to which at least two filter elements can be connected. The presence of an exhaust filter prevents the bioreactor from being contaminated from the outside of the bioreactor and also hampers cells from leaving the interior space of the rigid housing. The Y-connector greatly increases the versatility of the proposed bioreactor as different filters with different properties and even different materials can be used but do not need to be present for cases where they are not used.

A preferred embodiment specifies the first and/or second rigid support structure to be curved. In such a configuration, especially, when the first and/or second rigid support structure extend circularly around the stirrer shaft, the respective rigid support structure is arranged within a streamlined manner in direction of a circular flow of a fluid being stirred within the rigid housing.

One embodiment specifies the stirrer shaft to encompass impeller blades that are oriented perpendicular to the stirrer shaft which allows a liquid such as cell culture medium in the interior space of the rigid housing to be stirred. Blades being angled to a transversal plane of the stirrer shaft with an angle of 45° result in lower shear forces and higher mixing efficiency compared to a smaller angle of for example 30°.

Claims 2 relates to an embodiment of the single-use closed bioreactor with a lid consisting of multiple parts where at least a top part and a bottom part in combination form the cooling channel within the lid. This allows the formation of the cooling structure simply by providing two parts each one of which or both comprising a groove in an underside and/or an upper side that forms, when both parts are attached to each other, the cooling channel inside the lid. Such an embodiment simplifies the production of the cooling channel.

According to the preferred embodiment of claim 3, the channel ceiling and/or floor has an angled region which allows a condensate, condensed within the cooling channel, to flow in the direction of gravity along the angled region towards the channel inlet. This prevents an accumulation of condensate within the cooling channel thereby reducing the risk of condensate being carried out of the cooling channel with an exhaust gas stream through the channel outlet which might lead to exhaust filter blocking. Further, it enables the condensate to return to the bioreactor, thereby reducing the liquid loss.

Claim 4 specifies the channel inlet to have a distance to the wall of at least 15 % of the maximum width of the rigid housing and/or the cooling channel to have at least one turn. At a distance of the channel inlet to the wall of at least 15 % of the maximum width of the rigid housing the channel inlet is located nearby the stirrer shaft which results in immediate mixing of the condensate with a liquid stirred by the stirrer, as the condensate flows back in a well stirred region of the rigid housing. By providing the cooling channel with at least one, preferably multiple turns the cooling channel can be guided in different directions which allows an optimal usage of the lid area, the circumvention of lid installations such as other ports or probes and a further increase in cooling channel length. This further increase in length results in a further increased residence time of an exhaust gas passing the cooling channel and a larger available surface area for heat exchange and thus to a more efficient cooling of the exhaust gas.

In another embodiment according to claim 5 the horizontal section is located in a circular sector of the lid which assigns the cooling channel to a dedicated section of the lid. As this section is dedicated to the cooling structure only, no other installations such as ports need to be circumvented by the cooling channel.

Claims 6 to 8 relate to the number, location and construction of the bottom through port and specify the bottom through port to have a bottom through port channel. Such a bottom through port channel allows a fluid communication between the interior and exterior of the rigid housing. A bottom through port channel that extends from the bottom through port especially allows complete removal a liquid from the interior space of the rigid housing. Bottom through ports allow for a small working volume in a larger bioreactor without the effort of inserting long sensors or conduits from the lid. In another preferred embodiment the bottom through port may comprise a bottom through port sensor which allows the measurement of one or more bioreactor parameters.

Claims 9 and 10 relate to embodiments with sensor ports encompassed by the lid and/or closed bottom. Such embodiments allow the installation of sensors from different sides of the bioreactor. Due to the increased number of possible sensors which can be mounted to the bioreactor, such an embodiment also enables the use of sensors based on different measurement principles for the same parameter which allows an orthogonal measurement of said parameter resulting in an increased accuracy for the respective measured parameter.

Claim 11 refers to an embodiment with different functionalities of the lid being assigned to different sections of the lid. Such an embodiment enables a more intuitive and less error-prone handling of the bioreactor, as the various functions are not disorganized arranged on the lid which avoids for example an incorrect connection of external devices. Furthermore, an embodiment comprising a group of adapters being assigned to one section of the lid enables the specific design an adapter matching the group of adapters as a counterpart which further simplifies the handling of the bioreactor.

In another embodiment according to claim 12 the lid comprises stabilization ribs. Such ribs decrease the weight of the lid and thus of the bioreactor by simultaneously increasing the strength and rigidity of the lid which allows the rigid housing to withstand higher internal pressures.

Another teaching according to claim 13, which is of equal importance, relates to a bioreactor arrangement comprising a proposed single-use bioreactor, in particular pre-sterilized and arranged in a sterile packaging, preferably, wherein the bioreactor arrangement comprises the filter element connectable to the sterile connector of the second Y-connector port, in particular packaged together with the single-use bioreactor.

All explanations given with regard to the proposed single-use closed bioreactor are fully applicable.

Another teaching according to claim 14, which is of equal importance, relates to a use of a proposed single-use bioreactor, preferably, wherein the single-use bioreactor is in a use-state and connected to a cooling element, and/or, wherein the single-use bioreactor is used for cell and gene therapy or perfusion or mammalian cell culture.

All explanations given with regard to the proposed single-use closed bioreactor and the proposed bioreactor arrangement are fully applicable.

Another teaching according to claim 15, which is of equal importance, relates to a method of production of a proposed single-use bioreactor, preferably, wherein the first rigid support structure and/or the second rigid support structure is extruded, and/or, wherein the wall together with the closed bottom and/or the top lid part and/or the bottom lid part are injection molded and preferably glued together.

All explanations given with regard to the proposed single-use closed bioreactor, the proposed bioreactor arrangement and the proposed use are fully applicable.

In the following, embodiments of the invention are explained with respect to the drawing. The drawing shows in
- Fig. 1,: a) a schematic illustration of a proposed single-use closed bioreactor, and in b) an enlarged view of the bottom through ports with a sensor and a tube connection,
- Fig. 2,: a proposed single-use closed bioreactor in a sectional drawing in a side view, and in a sectional drawing in a top view,
- Fig. 3,: in a) the proposed single-use closed bioreactor shown in Fig. 2 in a sectional drawing in a side view, and an enlarged view of the cooling structure, in b) a schematic representation of the cooling structure consisting of two parts, and in c) a proposed single-use closed bioreactor in a top view of the lid,
- Fig. 4,: two variants of the bottom lid parts, a first variant in a), c) and e) and a second variant in b), d) and f),
- Fig. 5,: a) the proposed single-use closed bioreactor shown in Fig. 1 viewed from above, with the body hidden for better visibility, in b) an impeller in bottom view, and in c) an impeller in side view, and
- Fig. 6,: in a) two different sizes of the proposed bioreactor and an additional bioreactor with design parameters allowing a scaling of bioprocesses and b) a further variant of the proposed bioreactor.

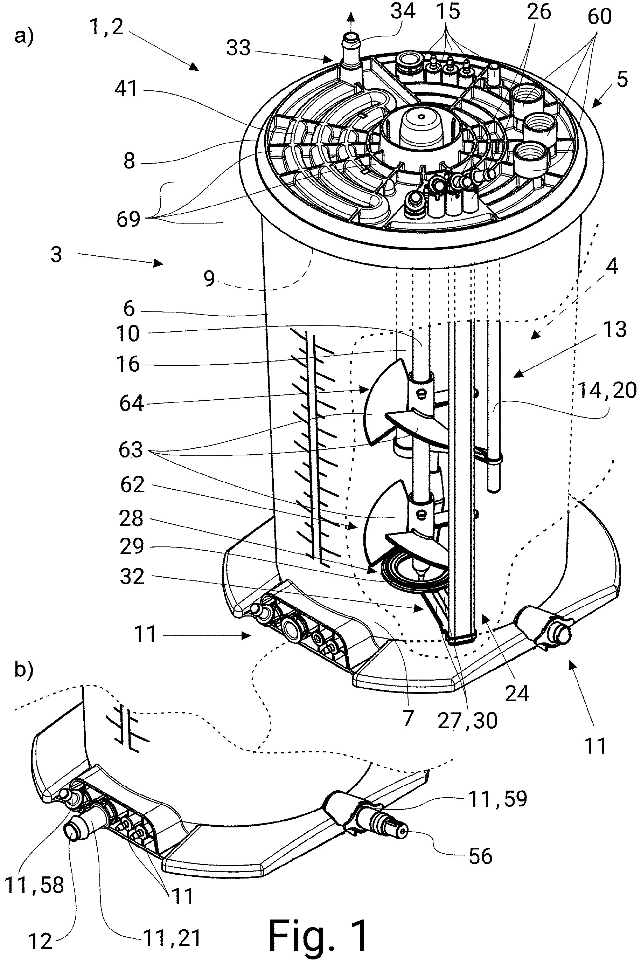

An exemplary embodiment of a proposed single-use closed bioreactor 1 for culturing, fermenting or processing a biomass is schematically shown in Fig. 1 a). The bioreactor 1 may be part of a bioreactor arrangement 2 during shipping and/or during use. The bioreactor 1 comprises a rigid housing 3 with an interior space 4 in which a liquid biological medium, in this case a biological reaction medium, consisting of the substances intended for a biotechnological process can be accommodated. The biological reaction medium may comprise microorganisms such as bacteria, yeasts, viruses or the like, mammalian cells, plant cells or the like, and a nutrient medium. In order to form a microbial or cellular product, the cells may be cultivated in the rigid housing 3 under specified conditions.

The rigid housing 3 has an inner height, an inner width and a volume smaller than 100 L. In some embodiments the volume is smaller than 80L, smaller than 50 L, smaller than 30 L or smaller than 20L. In some embodiments the volume is greater than 0,25 L, greater than 0,5 L, greater than 1 L, greater than 2 L or greater than 5 L. Furthermore, the rigid housing 3 comprises a lid 5 and a body, wherein the body comprises a wall 6, a top and a closed bottom 7 opposite to the top. The lid 5 has an upper surface 8 on its top directed to an environment outside the rigid housing 3 and a lower surface 9 on the other side of the lid 5, facing the closed bottom 7 of the rigid housing 3. The upper surface 8 and/or the lower surface 9 of the lid 5 may be flat and/or have a dome-like shape with a curvature either towards or away from the closed bottom 7.

The inner height of the bioreactor 1 is defined as the distance from the lowest point of the closed bottom 7 inside the rigid housing 3 upwards. If "upwards" cannot be identified, the direction of the rotational axis of the stirrer shaft 10 defines the direction of the height. The maximum inner height is the distance from the lowest point of the closed bottom 7 inside the rigid housing 3 upwards to the lower surface 9 of the lid 5 along the direction of the inner height. The inner width is defined as the distance of two opposite sides of the wall 6 inside the rigid housing 3 in a direction orthogonal to the direction of the inner height. Hence, the maximum width is the largest distance of two opposite sides of the wall 6 inside the rigid housing 3 in a direction orthogonal to the direction of the inner height.

The top of the body is irreversibly connected to the lid 5, preferably to the lower surface 9 of the lid 5, such that the top is closed by the lid 5. Here and preferably, the rigid housing 3 is formed by two separate parts with the body being one separate part and the lid 5 being another separate part. Both parts are then connected irreversibly with each other. Irreversibly connected according to the application means that two parts cannot be separated from each other without destroying one or both of the parts or an adhesive arranged between both parts. Hence, the lid 5 cannot be separated from the body without destroying the lid 5 and/or the body and/or an adhesive. Preferably, the lid 5 and the body can be separated via for example laser (un-)welding for recycling purposes, thereby destroying the connection between lid 5 and wall 6.

The above noted irreversible connection can, for example, be achieved by positive substance jointing such as gluing or welding, e.g. ultrasonic welding. Possible manufacturing processes for the lid 5 and body are known to the person skilled in the art. These include injection molding, 3D printing or machining.

Besides the rigid housing 3 consisting of at least, here and preferably exactly, two separate parts, the rigid housing 3 may also be formed by one single part where the lid 5 and body can only be distinguished by their definitions, namely that the lid 5 is the portion of the rigid housing 3 that covers the body and where both parts are inherently irreversibly connected. Such a rigid housing 3 may, for example, be manufactured by 3D printing. Materials for the lid 5 and body, and thus for the rigid housing 3, are known to the person skilled in the art. The materials of the lid 5 and the body preferably resist a sterilization process such as autoclaving, gamma irradiation, x-ray irradiation or beta particle irradiation. Preferably, the material of the lid 5 and body has minimum or no influence on the viability or productivity of the cells when cultivated within the bioreactor 1. Such an influence may, for example, be caused by leaching of substances from the respective material into a cultivation medium accommodated within the rigid housing 3.

As shown in Fig. 1 a) and b) the body comprises a bottom through port 11. The bottom through port 11 may be of a first type of bottom through port 11, which first type of bottom through port 11 allows a direct access and thus a material exchange from the outside to an inside of the body and thus from the outside to the interior space 4 of the rigid housing 3 or vice versa. Such types of bottom through ports 11 are known by a person skilled in the art and may, for example, be barb fittings, luer lock, luer slip, tri clamp adapters or the like. These types of bottom through ports 11 may be closed or be equipped with a sealing in order to prevent a material exchange from the interior space 4 with the outside of the rigid housing 3 and vice versa in general. The sealing provides the possibility of a material exchange when needed. Such a sealing may be a membrane or a valve, preferably a sample valve. The bottom through port 11 may comprise a sterile connector. t is also conceivable that the bottom through port 11 is permanently open but comprises a sealing being plugged to the bottom through port 11, e.g. a hose with a hose clamp. A bottom through port 11 may further be equipped with a connection for a tube 12 as shown in Fig. 1 b). The connection for the tube may be adapted to be used for perfusion, in particular alternating tangential flow filtration (ATF) perfusion. Preferably, a tube is permanently connected to the bottom through port 11 and reversibly closed to maintain sterility.

A second type of bottom through port 11 which may be present alternatively or additionally does not allow a material exchange but an exchange of measurable variables from the outside to the inside of the body and comprises a sensor connection through the wall 6 for an invasive sensor. Such a second type of bottom through port 11 can be seen on the right side of Fig. 1. Per definition, a contactless sensor that works through the wall 6 or bottom is not seen as a sensor using a through port. The sensor or multiple sensors using a bottom through port 11 of the second type may measure pH and/or conductivity and/or dissolved oxygen and/or glucose and/or temperature and/or redox potential and/or nitrogen and/or dissolved carbon dioxide and/or an amino acid and/or a protein concentration. The sensor is preferably a single-use sensor. A pH sensor positioned at the side of the single-use bioreactor 1 is advantageous in particular for cultures with microcarriers which may settle on optodes at the bottom if a stirrer is disabled.

The bioreactor 1 further comprises a stirrer shaft 10 which is arranged on the lid 5 of the bioreactor 1 and extends from the lid 5 into the interior space 4 of the rigid housing 3. The stirrer shaft 10 comprises a magnetic component, which is configured to interact with a magnetic drive located outside the rigid housing 3. Here, the magnetic component is attached to the stirrer shaft 10 and rotates with the stirrer shaft 10. Such a magnetic drive enables a contactless linkage of the drive to the stirrer shaft 10, thereby avoiding leakage problems and maintaining the sterility of the interior volume of the rigid housing 3 as the stirrer shaft 10 may completely be arranged within the rigid housing 3. As the magnetic drive allows the stirrer to turn without direct contact to the stirrer shaft 10 the rigid housing 3 remains gas-tight, permitting long continuous cultivation times up to e.g. 30 d, e.g. in perfusion cell cultures, and interior overpressures of up to 600 mbar.

The bioreactor 1 comprises a first rigid support structure 13 extending from the lid 5 into the interior space 4 of the rigid housing 3, wherein the first rigid support structure 13 comprises a, in particular rigid, first conduit 14. Rigid in the context of the first rigid support structure 13 means, that the first rigid support structure 13 is not flexible under bioprocess conditions, e.g. the first rigid support structure 13 withstands the current of a medium flow generated by the stirrer. Therefore, the first rigid support structure 13 differs to flexible hoses which considerably oscillate under bioprocess conditions, e.g. due to stirring of the cultivation medium. The first rigid support structure 13 may be any rigid structure on which the first conduit 14 may be attached to or in which the first conduit 14 may be included in, and which extends from the lid 5 into the interior volume.

This first rigid support structure 13 may be hollow with an open or closed interior end. For example, the first rigid support structure 13 may be a simple rigid rod extending from the lid 5 into the interior volume further comprising means to hold the first conduit 14. Preferably, the first rigid support structure 13 comprises or is made of the same material as the body and/or the lid 5.

The lid 5 comprises at least one first inlet port 15 connected to the first conduit 14 via which a fluid can be exchanged with the interior space 4 of the rigid housing 3 through the first conduit 14, e.g. a barb fitting, luer lock, luer slip, tri-clamp adapter or the like.

Here and preferably, the first conduit 14 is used for sampling liquid from the interior space 4. Alternatively and as will be described, the first conduit 14 may be used for introducing gas into the interior space 4. This is not the case in the shown embodiments.

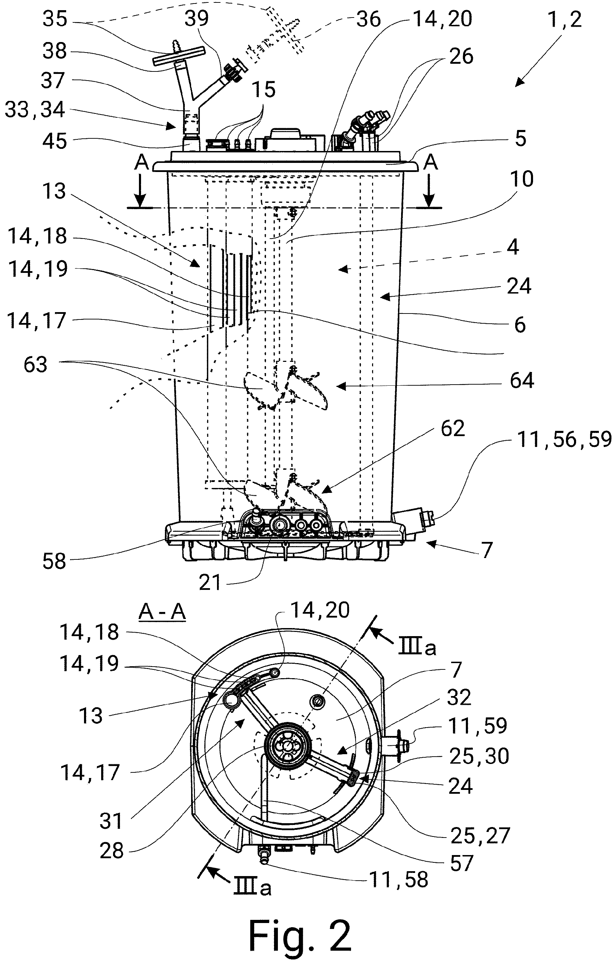

As shown in Fig. 2, the first rigid support structure 13 may comprise multiple first conduits 14 each connected to a first inlet port 15 of the lid 5. Here and preferably, the multiple first conduits 14 are rigid. Rigid in the context of conduits in general implies that the conduits are made of a non-flexible material such as rubber or the like, therefore differing to flexible hoses. The first rigid support structure 13 may comprise a one-piece conduit carrier 16 and at least two of the multiple first conduits 14 are formed by the one-piece conduit carrier 16. This embodiment is particularly easy to produce, for example by extrusion.

A respective first rigid support structure 13 is shown in the sectional drawing of Fig. 2 in a top view where the first rigid support structure 13 comprises a one-piece conduit carrier 16 with a front first conduit 17 at one end, a back first conduit 18 arranged on the opposite end and two middle first conduits 19 in between the front first conduit 17 and the back first conduit 18.

Additionally to the one-piece conduit carrier 16, the first rigid support structure 13 may comprise a separate first rigid conduit 20 arranged at a distance to the one-piece conduit carrier 16. The multiple first conduits 14 may have different diameters. For example, the front first conduit 17 has a larger diameter than the middle first conduits 19 and the back first conduit 18. This is especially advantageous for perfusion cell cultures. A perfusion cell culture typically utilizes a cell retention device such as a tangential flow filtration (TFF) or an alternating tangential flow filtration (ATF) device, and continuous media exchange to achieve and maintain high cell densities and viabilities over extended periods of time, e.g. up to 30 days. The cell retention device, which is in most of the cases arranged outside the bioreactor 1 and connected to the bioreactor 1 via a conduit, retains cells inside the bioreactor 1, while fresh media is added, and products of interest, waste products and spent (or depleted) media are continuously removed. This removal of cultivation media requires higher volumetric flows compared to the addition of substances to the volume. Therefore, in a perfusion cell culture using TFF the biomass may advantageously be removed from the front first conduit 17 as this conduit has the largest diameter making it beneficial for the higher volumetric flow rates required for the removal of cell culture medium. One or both of the middle first conduits 19 or a bottom through port 11 may be used to return retained cells from the TFF device back to the rigid housing 3 of the bioreactor 1.

As described above, a bottom through port 11, here an ATF bottom through port 21, with a connection for a tube 12 may be used for ATF perfusion cell cultures. In such an ATF perfusion cell culture the bottom through port 11 may be used for both, the removal of cell culture from the bioreactor 1 and for the return of retained cells back to the bioreactor 1. However, the ATF device may also be connected to any of the multiple first conduits 14 such as the front first conduit 17.

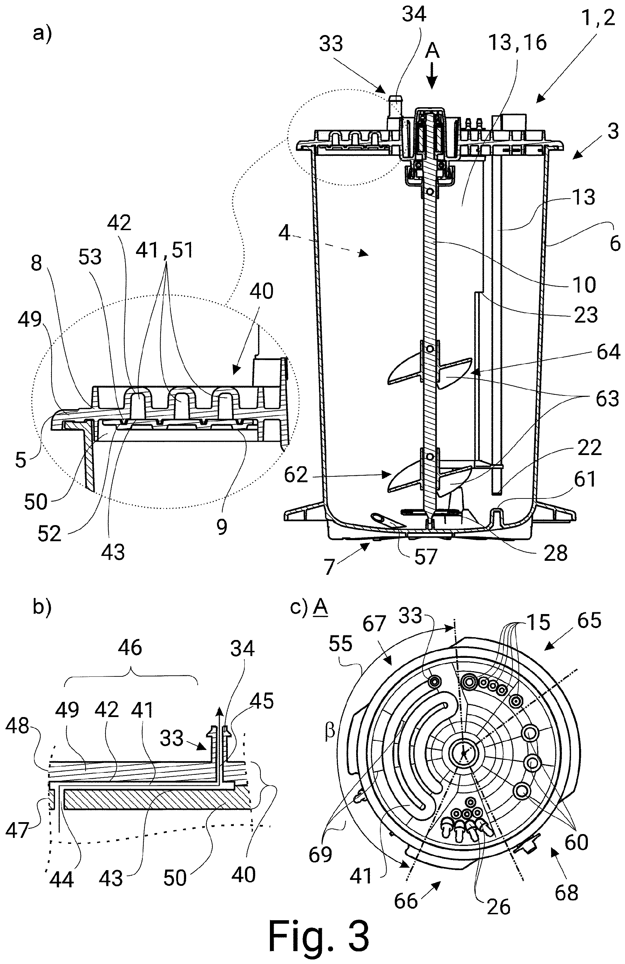

Here and preferably, the multiple first conduits 14 end at different inner heights, thus allowing an exchange of fluid at different inner heights. This may mean that each of the multiple first conduits 14 has a different length, or, in case of a one-piece conduit carrier 16 which is shaped in such a way that at least two of the multiple conduits are provided by the structure of the one-piece conduit carrier 16 itself, the one piece-carrier has multiple interior ends at different heights as shown in the side view of the bioreactor 1 of Fig. 3. Additional conduits ending at the same inner heights may be present in addition to the multiple first conduits 14.

Providing a first rigid support structure 13 with multiple first conduits 14 ending at different inner heights allows the removal of a liquid such as cell culture from the interior space 4 at different inner heights. Preferably, the height of one end of one of the multiple first conduits 14 differs to the height of one end of another one of the multiple first conduits 14 by more than 1 %, preferably more than 2 %, more preferably more than 5 %, and or at most 80 %, preferably at most 50%, more preferably at most 30 %, of the maximum inner height, preferably 5 % to 20 % of the maximum inner height. Preferably, at least one of the multiple first conduits 14 ends at an inner height of 0 % to 25 % and/or at least one at 25 % to 50 % and/or at least one at 50 % to 75 % and/or at least one at 75 % to 100 % of the maximum inner height. In Fig. 3, a lowest end 22 and a highest end 23 are well visible. At least one further inner end is located at the end of the one-piece conduit carrier 16 slightly above the lowest end 22.

As shown in Fig. 1 a), the bioreactor 1 may comprise a second rigid support structure 24 extending from the lid 5 into the interior space 4 of the rigid housing 3. Here and preferably, the second rigid support structure 24 comprises a second conduit 25, and the lid 5 comprises at least one second inlet port 26 via which a fluid can be exchanged with the interior space 4 of the rigid housing 3 through the second conduit 25. Anything said with regard to the first rigid support structure 13 may be true additionally or alternatively for the second rigid support structure 24. Preferably, the second rigid support structure 24 comprises or is made of the same material as the body and/or the lid 5. In an embodiment not shown, the first rigid support structure 13 has the features of the second rigid support structure 24, possibly without another rigid support structure being present, i.e. the numbering "first" and "second" is only for better understanding.

The first rigid support structure 13 and the second rigid support structure 24 may be located within a radial distance to a rotational axis of the stirrer shaft 10 differing by at most 20 % of the inner width, preferably at most 15 % of the inner width, more preferably at most 10 % of the maximum inner width. Here and preferably, the first rigid support structure 13 and the second rigid support structure 24 are located on the lid 5 at the same radial distance from the rotational axis of the stirrer shaft 10 to the wall 6 of the rigid housing 3.

The bioreactor 1 preferably comprises a tube arrangement comprising at least one, in particular several, tubes connected to one or more ports of the bioreactor 1, e.g. the first inlet ports 15 and/or the bottom through port 11 and/or the second inlet ports 26. It is preferably the case that every port of the bioreactor 1 is connected to a tube or closed by a plug added by the manufacturer which preferably is not removed during normal use by a user. The tubes may end in a sterile connector.

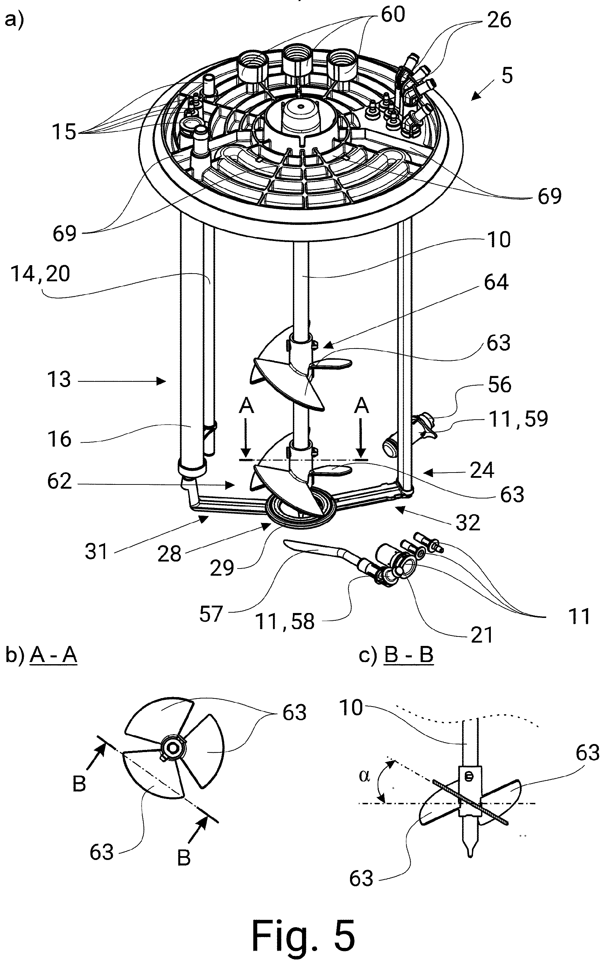

As shown in the cross-section at the bottom of Fig. 2, the second rigid support structure 24 may comprise a first gas conduit 27 for introducing a first gas into the interior space 4 of the rigid housing 3 and an aeration device 28 which has a first group of holes (not shown) through which the first gas can be released into the interior space 4, whereby the first group of holes are connected to the first gas conduit 27. Such a configuration enables an aeration of the bioreactor 1 within the rigid housing 3 with the first gas. The aeration device 28 may comprise a central portion, in particular central ring 29, which comprises the first group of holes and/or the second group of holes (best visible in Fig. 5).

Here and preferably the second rigid support structure 24 further comprises a second gas conduit 30 for introducing a second gas into the interior space 4 of the rigid housing 3. The aeration device 28 may comprise a second group of holes through which the second gas can be released into the interior space 4 and the second group of holes is connected to the second gas conduit 30. Such a configuration allows an aeration of the bioreactor 1 with two different kinds of gases.

The first and the second gas may be of the same type of gas, here and preferably, the first gas and second gas are different. It is conceivable that the first and/or second gas may be oxygen, carbon dioxide, nitrogen, air or the like. Preferably the first gas may be an oxygen-containing gas such as oxygen or air, providing oxygen required for the metabolism of cells being cultivated in the bioreactor 1. The second gas may, for example, be a carbon dioxide-containing gas, preferably with a carbon dioxide concentration higher than air and more preferably essentially pure carbon dioxide, which allows the adjustment of the pH value of the cultivation medium.

The first and second group of holes may differ in their geometric parameters. Such parameters may comprise the diameter of the holes and/or the orientation of the holes. The first group of holes may have a diameter of at most 0.2 mm, preferably of 0,005 to 0.2 mm, more preferably of 0.02 to 0.18 mm, here of 0.15 mm and/or a cross-sectional area of at most 0.03 mm², preferably of 0.00002 to 0.03 mm², more preferably of 0.0003 to 0.025 mm². The second group of holes may have a diameter larger than 0.2 mm, preferably of 0.25 to 4.0 mm, more preferably of 0.5 to 1.0 mm, here of 0.8 mm, and/or a cross-sectional area larger than 0.03 mm², preferably of 0.05 to 12.0 mm², more preferably of 0.2 to 0.8 mm². It is conceivable that the first group of holes is arranged on a top surface of the aeration device 28 facing towards the top of the body while the second group of holes is arranged on a bottom surface of the aeration device 28 facing towards the closed bottom 7 of the body or vice versa. However, in another configuration both group of holes may be arranged on the same surface of the aeration device 28.

The first group of holes and/or the second group of holes may be formed by injection molding during a manufacturing process of the aeration housing. Preferably, the first group of holes and/or the second group of holes are introduced into the aeration housing by drilling, e.g. by laser-drilling. The aeration device 28 is located here and preferably below an impeller comprised by the stirrer shaft 10 which will be explained later in more detail. Here and preferably, the aeration device 28 is arranged around the stirrer shaft 10 and preferably coaxially thereto.

As an alternative to the first conduit 14 being used for sampling, it may be the case that the first gas conduit 27 is connected to the first conduit 14 and the aeration device 28 is connected to the first rigid support structure 13. The first rigid support structure 13 and the first conduit 14 may then be used for introducing gas into the interior space 4. It may also be the case that the first rigid support structure 13 comprises conduits, comprising the first conduit 14, for sampling and for introducing gas, in particular the first gas and the second gas via at least two conduits, into the interior space 4 via the aeration device 28, in which case the second conduit 25 may not be present.

The cross-section in Fig. 2 shows that the aeration device 28 may be structurally connected with the first and the second rigid support structure 24. Structurally connected means that a force can be transferred between the respective structures.

The aeration device 28 may be structurally connected with the first rigid support structure 13 by means of a first extension 31 which extends from the aeration device 28 towards the first rigid support structure 13 (Fig. 5). Here and preferably, the first extension 31 is an integral part of the aeration device 28 and mounted to the first rigid support structure 13. Due to their structural connection the first rigid support structure 13 and the second rigid support structure 24 support each other which reduces the stress on each single rigid support structure leading to a reduced deformation of both structures 13, 24. Such a stress and deformation may for example be caused by a flow of the cultivation medium, e.g. due to a stirred cultivation medium.

The aeration device 28 may be connected to the second rigid support structure 24 via a second extension 32. Here and preferably, the second extension 32 is an integral part of the aeration device 28 and mounted to the second rigid support structure 24.

Fig. 5 shows the single-use bioreactor 1 with the wall 6 and closed bottom 7 missing. The bottom through ports 11 show where the wall 6 would be. Here and preferably, the second extension 32 comprises a part of the first gas conduit 27 and/or a part of the second gas conduit 30.

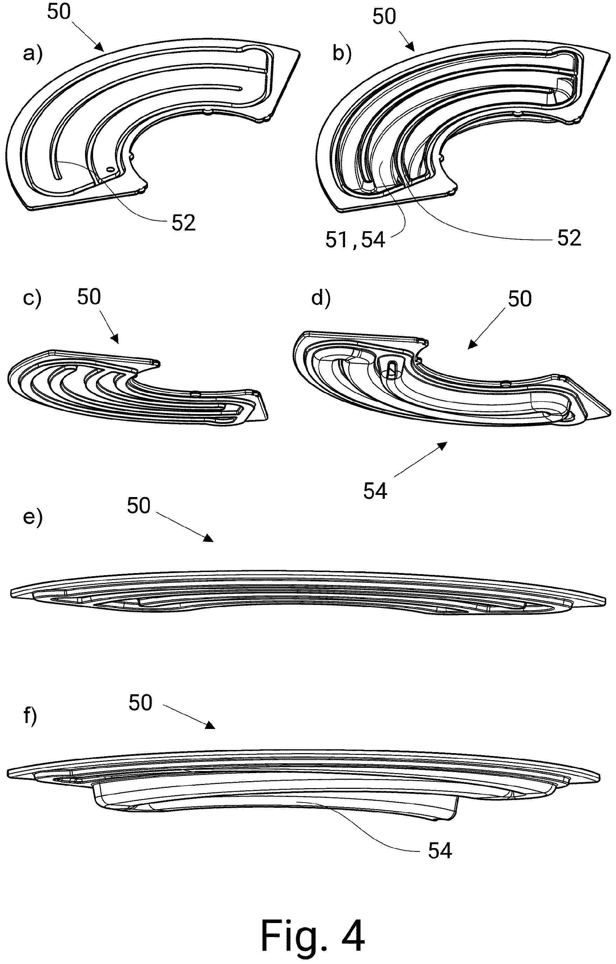

As can be seen in Figs. 3 and 4, for example, here and preferably the rigid housing 3 comprises an exhaust port 33 via which an exhaust gas can be removed from the rigid housing 3. An exhaust port 33 enables the removal of an exhaust gas such a gaseous component, which may either be produced by the cultivation of cells and/or by an aeration of the bioreactor 1, thereby avoiding a pressure built-up. Here and preferably the exhaust port 33 is equipped with an exhaust adapter 34 that allows the connection to other devices such as filter elements 35 or exhaust gas analysis devices for qualitative and/or quantitative determination of a gaseous component in the exhaust gas such as water vapor, oxygen, carbon dioxide or specific components produced during the cultivation process.

Preferably, the exhaust port 33 is connected or connectable to at least two filter elements 35 at once through each one of which or through both the exhaust gas can be filtered. Each of the at least two filter elements 35 comprises a filter material through which the exhaust is filtered. Preferably, the filter materials of the at least two filter elements 35 are different filter materials. It may be the case that the bioreactor 1 is shipped as part of a bioreactor arrangement 2 with one irreversibly connected filter element 35 and at least one second filter element 36.

As shown Fig. 2 a) the exhaust may be connected to a Y-connector piece 37 with a first Y-connector port 38 and a second Y-connector port 39 to which the at least two filter elements 35 can be connected. Here and preferably, a first filter element 35 is connected to the first Y-connector port 38 while the second Y-connector port 39 comprises a sterile connector for connecting a second filter element 36. A sterile connector is any type of closing of the second Y-connector port 39 that on the one hand keeps the second Y-connector port 39 sealed and sterile, and on the other hand allows a sterile connection of the second filter element 36 to the second Y-connector port 39.

Preferably, a membrane filter, such as a PTFE filter is connected to the first Y-connector port 38. In another preferred embodiment, a post integrity testable filter, in particular with a high tolerance for water vapor, is connected to the second Y-connector port 39.

Here and preferably it is proposed that the first and/or second rigid support structure 13, 24 is curved. Preferably, the first rigid support structure 13 and/or the second rigid support structure 24 is curved around the stirrer shaft 10 and more preferably the first rigid support structure 13 and/or the second rigid support structure 24 extends circularly around the stirrer shaft 10 as illustrated in the cross-section in Fig. 2. This means that the first and/or second rigid support structure 13, 24 extends in top view along a circular path of a single radius around the stirring rod. Such a curved support structure causes less shear and is therefore advantageous in particular for low shear applications like cell and gene therapy applications.

As shown in Fig. 2 the first rigid support and the second rigid support structure 24 both extend circularly around the stirrer shaft 10 and both preferably have the same radial distance from the rotational axis of the stirrer shaft 10 to the wall 6 of the rigid housing 3 which advantageously reduces shear forces during a mixing of the cultivation medium.

As shown in Fig. 3, in one embodiment, the lid 5 comprises a cooling structure 40 for cooling an exhaust gas leaving the bioreactor 1. This cooling structure 40 is part of an exhaust port 33 which enables the removal of gaseous components, which may either be produced by the cultivation of cells and/or by aeration of the bioreactor 1 thereby avoiding a pressure built-up within the rigid housing 3. The exhaust port 33 may be completely or partially as described previously. However, any type of exhaust port 33 can be combined with this embodiment.

The cooling structure 40 comprises a cooling channel 41 with a channel ceiling 42 and a channel floor 43, whereby the channel floor 43 and the channel ceiling 42 are both part of the lid 5. The cooling channel 41 allows the transfer of heat from the cooling channel 41 to the upper surface 8 of the lid 5 and thus serves as a heat exchanger enabling the cooling of the exhaust gas that passes the cooling channel 41. As illustrated in the enlarged view of cooling structure 40 in Fig. 3 b), the cooling channel 41 has a channel inlet 44 at the lower surface 9 of the lid 5 which connects the interior space 4 of the rigid housing 3 with the cooling channel 41. The cooling channel 41 further comprises a channel outlet 45 at the upper surface 8 of the lid 5 which connects the cooling channel 41 with an environment outside the rigid housing 3. Thus, an exhaust gas may pass from the interior space 4 through the channel inlet 44, the cooling channel 41 and the channel outlet 45 from the interior space 4 of the rigid housing 3 to the environment outside the rigid housing 3.

The channel inlet 44 is connected to the channel outlet 45 via the cooling channel 41 such that the exhaust gas can be removed from the interior space 4 through the channel inlet 44, the cooling channel 41 and the channel outlet 45. The cooling channel 41 has a horizontal section 46 which is at least partially oriented horizontally within the lid 5. "At least partially horizontal" means that the cooling channel 41 does not follow the shortest possible path through the lid 5 and instead is substantially longer than that shortest connection, e.g. at least twice, preferably at least four times as long. Furthermore, the cooling channel 41 preferably has an insulating lower barrier 47 between the channel floor 43 and the lower surface 9 of the lid 5, preferably with a thickness of 2.0 mm to 3.5 mm, preferably 2.5 mm to 3 mm. Accordingly, the cooling channel 41 has an insulating upper barrier 48 between the channel ceiling 42 and the upper surface 8 of the lid 5, preferably with a thickness of 1 mm to 3 mm, preferably 1.5 mm to 2.5 mm. Independently of the named sizes, the insulating lower barrier 47 is preferably at least 10 %, preferably at least 20 %, thicker than the insulating upper barrier 48.

The cooling structure 40 allows a vapor of a liquid, preferably water vapor being present in the exhaust gas, to be condensed, which can be achieved by lowering the temperature of the exhaust gas below the condensation temperature in the cooling channel 41. This cooling can be achieved by a transfer of heat from the cooling channel 41 through the insulating upper barrier 48, when the upper surface 8 of the lid 5 within the cooling structure 40 has a lower temperature compared to the horizontal section 46. This lower temperature can either be obtained by a lower temperature of the environment outside the rigid housing 3 around the cooling section or, preferably, by a cooling element being arranged on upper surface 8 within the region of the cooling structure 40. Preferably, this cooling element matches the structure of the upper surface 8 of the lid 5 within the cooling structure 40 thereby providing a high contact area and thus a high heat transfer. For example, a bottom surface of the cooling element may be the inverse of a top surface of the channel. The cooling element may preferably be actively cooled, e.g. by a cooling liquid passing the cooling element or by means of a peltier element. The bioreactor arrangement 2 may comprise the cooling element.

Furthermore, an appropriate dimension of the insulating upper barrier 48 and the insulating lower barrier 47 allows an optimal cooling of the cooling channel 41 with only very little extraction of heat from the interior space 4 due to low or no heat transfer through the lower layer. This eliminates the need for an appropriate heat control of the bioreactor 1 which compensates for a heat loss through the cooling structure 40.

Here and preferably and as illustrated in Fig. 3 a) and schematically in Fig. 3 b), the lid 5 may consist of multiple parts with at least a top lid part 49 comprising the channel ceiling 42 and with a bottom lid part 50 comprising the channel floor 43, where the bottom lid part 50 is attached to the top lid part 49 so that the cooling channel 41 is formed. Here and preferably, the bottom lid part 50 is glued to the top lid part 49. Here and preferably the channel is formed by exactly two lid 5 parts. Fig. 4 shows two variants of the bottom lid part 50, a first variant in a), c) and e) and a second variant in b), d) and f). Fig. 4 a) and b) are views from above, c) and d) views from below and e) and f) views from the side and slightly below.

The top lid part 49 may comprise a groove 51 on an underside of the top lid part 49 as shown in the enlarged view of cooling structure 40 of Fig. 3 a). The bottom lid part 50 may comprise a flat surface on an upper side of the bottom lid part 50 (first variant, Fig. 4). When the top lid part 49 is attached to the bottom lid part 50 by attaching the upper side of the bottom lid part 50 to the underside of the top lid part 49 as illustrated in the enlarged view of cooling structure 40 of Fig. 3 a), the flat surface and the groove 51 form the cooling channel 41 of the cooling structure 40. Alternatively, the bottom lid part 50 may comprise a groove 51 (second variant). Here and preferably, the top lid part 49 comprises a groove 51 and the bottom lid part 50 comprises a groove 51 arranged opposite to the groove 51 of the top lid part 49. When the top lid part 49 and the bottom lid part 50 are attached to each other as described above, the groove 51 of the top lid part 49 and the groove 51 of the bottom lid part 50 form the cooling channel 41 of the cooling structure 40.

As also shown in the enlarged view of cooling structure 40 in Fig. 3 a) and in Fig. 4 a) - f) the bottom lid part 50 and/or the top lid part 49 may comprise a sealing groove 52 and/or a sealing protrusion 53 along the length of the channel in order to provide a sealing of the cooling channel 41 along the sides. The sealing protrusion 53 can be seen in the enlarged view in Fig. 3 a).

The channel ceiling 42 and/or the channel floor 43 may have an angled region 54 which is angled to a plane being perpendicular to the direction of gravity (Fig. 4 b), d), f)). The angle between the plane and the channel ceiling 42 and/or the channel floor 43 may be at least 1°, preferably at least 2°, more preferably at least 3°. Including an angle in the channel ceiling 42 and/or the channel floor 43 allows a condensate, condensed within the cooling channel 41, to flow towards a predefined area of the cooling channel 41, preferably the condensate can flow towards the channel inlet 44.

The channel inlet 44 may have a distance to the wall 6 of at least 10 %, preferably at least 15 %, more preferably at least 20 % and even more preferably between 20 % and 30 % of a maximum width of the housing. In such a case the channel inlet 44 is arranged in the proximity of the stirring rod which allows the condensate to flow directly back into a well-stirred region of a stirred fluid during cultivation. This results in an immediate mixing of the condensate with the stirred cultivation medium.

Here and preferably as shown for example in Fig. 3, the horizontal section 46 may comprise at least one turn, preferably at least two turns, more preferably at least three turns. The cooling channel 41 may as shown for example in the top view of the lid 5 in Fig. 3 extend horizontally in a snake-like pattern within the cooling structure 40 which results in an increased length of the cooling channel 41 and therefore a large surface area for heat transfer.

The horizontal section 46 may be located in a circular sector 55 of the lid 5 as shown in the top view of the lid 5 of Fig. 3. This circular sector 55 is defined by a partial area of a circular surface that is bounded by two circle radii. The angle β of the circular sector 55 may be smaller than 180°, preferably smaller than 150°, more preferably smaller than 120°, more preferably smaller than 100°, around the center axis of the stirrer shaft 10. A cooling channel 41 which extends in a snake-like pattern within a defined section of the lid 5 as shown in the top view of the lid 5 of Fig. 3 increases the surface area available for an exchange of heat while simultaneously assigning the cooling channel 41 an own segment of the lid 5 in which the cooling channel 41 can extend without having to be passed around other elements such as connection ports. Segregating the lid 5 into circular sectors 55 also enables putting the cooling element on the cooling channel 41 without interfering with other functions of the lid 5 and cooling the whole channel.

As already mentioned above the bottom through port 11 may be located in the wall 6 of the body. Preferably, the bottom through port 11 is located in the wall 6 of the body at a height smaller than 25 %, preferably smaller than 15 %, more preferably smaller than 10 % of the maximum inner height.

As shown in Fig. 1 b) the bottom through port 11 may comprise a bottom through port sensor 56 for determination of one or more parameters of the bioreactor 1. The bottom through port sensor 56 may be irreversibly connected to the bottom through port 11.

As shown in Fig. 5 a) here and preferably the bottom through port 11 may comprise a bottom through port channel 57 via which a fluid can be exchanged with the interior space 4 of the rigid housing 3. Preferably, the bottom through port channel 57 extends from the bottom through port 11 into the interior space 4, in particular to the closed bottom 7 of the body, even more preferably to the or a lowest point of the closed bottom 7 as shown in Fig. 5 a). By usage of the bottom through port channel 57 it may be possible to drain the bioreactor 1 to a volume of less than 100 ml, preferably less than 50 ml, more preferably less than 30 ml, and/or less than 1 % of the volume, preferably less than 0.5 % of the volume. The volume is the complete volume defined by the interior space 4. Preferably, the single-use bioreactor 1 also comprises a defined maximum working volume with a defined maximum working height. In that case, by usage of the bottom through port channel 57 it may be possible to drain the bioreactor 1 to a volume of less than 1 % of the maximum working volume, preferably less than 0.5 % of the maximum working volume.

As shown in Figs. 1 a) and 5 a) the body may also comprise multiple bottom through ports 11. As illustrated in Fig. 5 a) a first bottom through port 58 may comprise a bottom through port channel 57 and a second bottom through port 59 may comprise a bottom through port sensor 56.

Figs. 1 a) and 5 show that the lid 5 may comprise a lid sensor port 60 configured to receive a sensor for determination of one or more parameters of the bioreactor 1.

Here and preferably, and as shown in Fig.3 a), the closed bottom 7 may comprise a bottom sensor port 61 configured to receive a bottom sensor for measuring one or more parameters of the bioreactor 1. Preferably, the bottom sensor port 61 contains one or more optodes, in particular immobilized dyes, within the interior space 4 of the rigid housing 3 which are configured to interact non-invasively through the bottom sensor port 61 with the bottom sensor being located outside the body in the region of the bottom sensor port 61. An optode may be usable for a dissolved oxygen measurement and/or an optode may be usable for a pH measurement. The bottom sensor port 61 is therefore not a bottom through port 11.

As shown in Fig. 1 a) the stirrer shaft 10 may comprise a first impeller 62 with blades 63 for stirring a fluid in the rigid housing 3. The first impeller 62 may for example be a three blade 63 impeller, or a Rushton impeller or an Elephant ear impeller. The blades 63 are oriented perpendicular to the stirrer shaft 10. Preferably, the blades 63 are angled to the transversal plane of the stirrer shaft 10. This angle α is preferably between 20° and 80°, more preferably between 30° and 50°, for example 30° or 45°. The stirrer shaft 10 may also comprise multiple impellers. Here and preferably, the stirrer shaft 10 comprises a second impeller 64. The stirrer shaft 10 may be able to turn at up to 1500 rpm, preferably 2000 rpm, measured when the volume is filled with water, for at least 1 hour, preferably at least 1 day, more preferably at least 30 days.

According to one embodiment it is proposed, that different functionalities of the lid 5 are assigned to different sections of the lid 5. A functionality of the lid 5 may be any functionality that is essential for the cultivation of cells within a bioreactor 1. Here and preferably and as shown in Fig. 3, one or more first inlet port 15 is assigned to a first lid section 65 and/or one or more second inlet port 26 is assigned to a second lid section 66 and/or the exhaust port 33, in particular with the cooling structure 40 is assigned to a third lid section 67 and/or the lid sensor port 60 is assigned to a fourth lid section 68, and, at least two, preferably at least three, more preferably all, of the first lid section 65 and the second lid section 66 and the third lid section 67 and the fourth lid section 68 do not overlap. Therefore, here and preferably, the first lid section 65 is assigned to feeding functionalities and/or the second lid section 66 is assigned to gassing functionalities and/or the third lid section 67 is assigned to degassing functionalities with the exhaust port 33 preferably located more or less at an opposite side of the gassing functionalities and/or the fourth lid section 68 is assigned to sensor functionalities. Here and preferably, each lid 5 section is assigned to one circular sector 55 of the lid 5. However, each lid 5 section may alternatively be assigned to its own ring-shaped sector extending around the center of the lid 5.

It should be noted that the sensor ports are preferably closed by the manufacturer such that the bioreactor 1 can be sterilized as a whole. Therefore, the sensor or sensor head is preferably introduced by the manufacturer and not by a user.

According to one embodiment it is proposed, that the upper surface 8 of the lid 5 comprises stabilization ribs 69 for stabilizing the lid 5 which extend outwards from the upper surface 8 as shown in Figs. 1 a) and 5 a) and/or that the lower surface 9 of the lid 5 comprises stabilization ribs 69 for stabilizing the lid 5 which extend in the interior space 4 of the rigid housing 3 (not shown). By introducing stabilization ribs 69 in a surface of the lid 5 the weight of the lid 5 can be reduced and lid 5 material can be saved which results in lower manufacturing costs. Preferably, the lid 5 upper surface 8 and/or the lid 5 lower surface 9 comprises a first set of stabilization ribs 69 extending continuously or discontinuously radially from the center of the lid 5 and a second set of stabilization ribs 69 which interconnect the first set of stabilization ribs 69 with each other.

Another teaching which is of equal importance relates to a bioreactor arrangement 2 comprising the proposed single-use bioreactor 1 in particular pre-sterilized and arranged in a sterile packaging. Preferably, the bioreactor arrangement 2 comprises the second filter element 36 connectable to the sterile connector of the second Y-connector port 39, in particular packaged together with the single-use bioreactor 1. Additionally or alternatively, the bioreactor arrangement 2 may comprise during use the cooling element and/or a heating blanket and/or a cooling blanket and/or a magnetic drive driving the stirrer shaft 10. The heating blanket and/or the cooling blanket, which may be the same blanket, may be used to add or remove heat through the wall 6 of the bioreactor 1. The wall 6 may therefore be another functional area of the bioreactor 1, in addition to the sections of the lid 5. The heating and/or the cooling blanket may comprise a cutout for access to the bottom through port 11 or ports. The bioreactor arrangement 2 may also comprise a filter heater for the first filter element 35 and/or the second filter element 36. In particular behind the cooling channel 41, heating the filter 35, 36 may be beneficial.

Another teaching which is of equal importance relates to a use of the proposed single-use bioreactor 1. Preferably, the single-use bioreactor 1 is in a use-state and connected to the cooling element, and/or, the single-use bioreactor 1 is used for cell and gene therapy or perfusion or mammalian cell culture or bacterial or microbial processes.

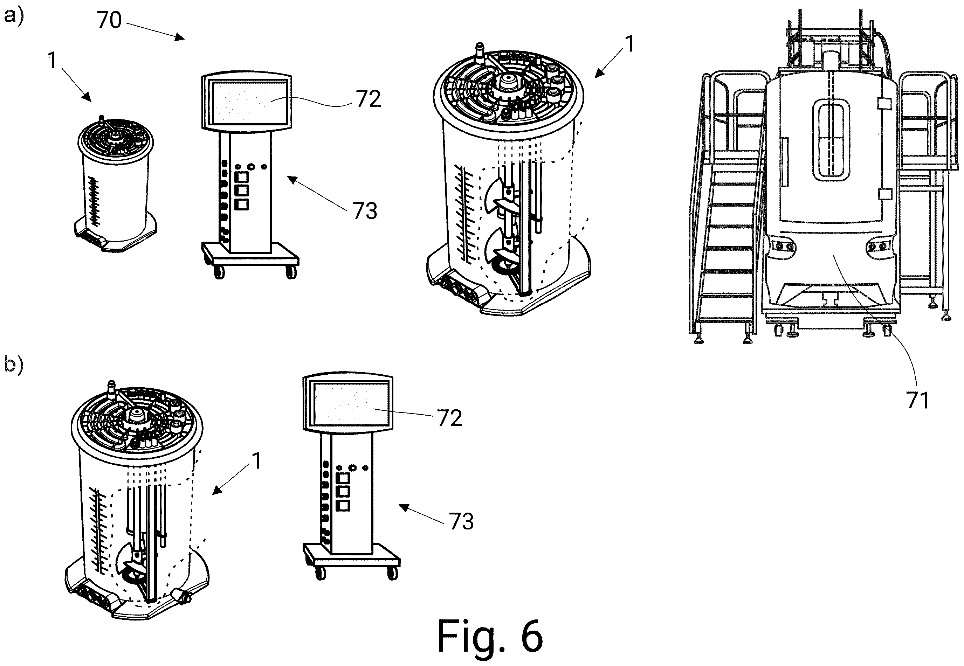

Another teaching which is of equal importance relates to a bioreactor series 70 comprising at least two variants of a single-use bioreactor 1 as described and at least one additional bioreactor 71 with a different volume, in particular a volume greater than 100 L. Fig. 6 shows in a) two sizes of the proposed single-use bioreactor 1 and an additional bioreactor 71. Fig. 6 shows in b) a variant of the second size of the bioreactors 1 in Fig. 6 a). The variants of the described bioreactor 1 may comprise a variant for the culture of mammalian cells (E-variant) and/or a variant optimized for perfusion processes (P-variant) and/or a variant optimized for cell and gene therapy related processes (C-variant). A key advantage of the proposed single-use bioreactor 1 is that it is possible to produce these variants while keeping most of the single-use bioreactor 1 the same. Producing different variants from a product is advantageous for the manufacturer and for the buyer who is able to use many known features for different processes. Mammalian processes in particular are often scaled. It may therefore be a requirement to use the proposed single-use bioreactor 1 for processes that are to be scaled up or down. It is therefore proposed to integrate the single-use bioreactor 1 into a series of at least two, preferably at least three, more preferably at least four, bioreactors 1 of different volumes. The bioreactor series 70 may comprise a bioreactor 1 of a volume greater than 500 L, and/or greater than 1000 L. The bioreactor series 70 may also comprise a bioreactor 1 smaller than 10 L, and/or smaller than 1 L. These may be variants of the proposed single-use bioreactor 1. It may be the case that the bioreactor series 70 comprises at least two of the proposed single-use bioreactors 1 with different volumes.

Everything said for the E-variant may be true for a further bacterial or microbial variant or the E-variant may be used for bacterial and/or microbial processes.

The variants may be mostly derived by not using, in particular disabling, some of the present features. However, it may be the case that in some variants, in particular the C-variant, a different impeller is used than in the E- variant and/or the P-variant.

The bioreactor 1 or bioreactors 1 with a volume greater than 100 L and a variant of the proposed single-use bioreactor 1, in particular the E-variant, may comprise a ratio of inner height to diameter measured at 50 % of the inner height from 1.7 to 1.9, preferably 1.8. The ratio of maximum filling height to diameter measured at 50 % may be between 1.2 and 1.4, preferably 1.3. The ratio of an impeller diameter to the diameter measured at 50 % of the inner height may be between 0.3 and 0.5, preferably 0.35 to 0.45.

In particular with regard to the proposed use and the proposed bioreactor series 70, but also in general, the single-use bioreactor 1 may be adapted to be used and preferably may be used to form the E-variant by connecting a gas to only the second gas conduit 30 and therefore the second group of holes and not to the first gas conduit 27 and the first group of holes. It may be the case that the cooling element is not used. It may be the case that the ports for perfusion are not used and possibly plugged. It may be the case that the bottom through port 11 of the second type for measuring pH is not used and possibly plugged instead of comprising a pH sensor.

The single-use bioreactor 1 may be adapted to be used and preferably may be used to form the P-variant by connecting the cooling element and/or a filter to the sterile connector and/or by connecting of ATF or TFF perfusion modules to ports of the bioreactor 1, in particular at least one port of the lid 5 and at least one bottom though port. Both groups of holes may be used. The bottom through port 11 of the second type for measuring pH may be used.

To form the C-variant the single-use bioreactor 1 may comprise a different type of stirrer, in particular an elephant ear impeller. A filter may be connected to the sterile connector. No cooling element may be used. Both groups of holes may be used. The bottom through port 11 of the second type for measuring pH may be used.

The bioreactor series 70 may comprise a software with a graphical user interface 72 which can be used for two or more, in particular all, of the variants and/or at least one variant and at least one, in particular all, of additional bioreactor 71. It may be possible to choose the variant and/or additional bioreactor 71 in the software. It may be possible to transfer a recipe from one variant, in particular the E-variant, to an additional bioreactor 71 in the software with support of the software. The graphical user interface 72 may comprise a layout of ports. Connections to the ports may also be visualized.

The tube arrangements of different variants may comprise different types of tubes, in particular tube materials. For example, one variant, in particular the C-variant, may comprise a medical-grade tube.

Another teaching which is of equal importance relates to a computer program product with instructions which, when the instructions are executed by a computer, in particular a bioreactor control unit 73, cause a computer, in particular the bioreactor control unit 73, to perform the functions of a software which is adapted to be used for two or more, in particular all, of the variants and/or at least one variant and at least one, in particular at least two, additional bioreactor 71 or additional bioreactors 71 of the bioreactor series 70.

Another teaching which is of equal importance relates to a bioreactor control unit 73 adapted to be used for controlling two or more, in particular all, of the variants and/or at least one variant and at least one, in particular at least two, additional bioreactor 71 or additional bioreactors 71 of the proposed bioreactor series 70, wherein the bioreactor control unit 73 has at least one difference between the variants and/or a variant and the additional bioreactor 71 saved in a memory. Using a software to control several bioreactors 1 of different variants and/or sizes makes it easier for a user to learn to use the software. It may also be the case that the bioreactor control unit 73 uses this saved difference to support a user with transferring a recipe from one variant to the additional bioreactor 71. Scaling recipes from small batches to big batches is often a very central problem of product development. A series of bioreactors 1 scaling well and with software supports is therefore advantageous. The bioreactor control unit 73 may comprise the graphical user interface 72.

Another teaching which is of equal importance relates to a method of production of a single-use bioreactor 1, preferably, wherein the first rigid support structure 13 and/or the second rigid support structure 24 is extruded, and/or, wherein the wall 6 together with the closed bottom 7 and/or the top lid part 49 and/or the bottom lid part 50 are injection molded and preferably glued together.

Also relevant is a method of production of the bioreactor series 70, wherein at least one part of the bioreactor 1 is identical for at least two variants and produced at the same production line.

The single-use bioreactor 1 may be assembled during production by connecting several parts. The single-use bioreactor 1 may be sterilized after the assembly. Many of the parts have already been described and can be seen in the figures. The following focuses on the details of the production.

The aeration device 28 may comprise at least, preferably exactly, three parts. One part may comprise part of the central ring 29, part of the second extension 32 and the first extension 31. Another part may comprise part of the second extension 32. A third part may comprise part of the central ring 29. In particular, the first and/or second gas conduit 30 may run through the second extension 32 and the central ring 29 and may be formed by gluing together the three parts. Prior to the connection of the three parts the conduits may be open and then closed by the connection of the parts. The first extension 31 which may be free of conduits may then be formed by only one part.

The first and/or the second rigid support structure 24 may be glued to the lid 5, in particular the top lid part 49, and to the aeration device 28, in particular the respective extension. At least one of, some, or here and preferably all, of the ports named herein may be glued to the wall 6 or the lid 5 respectively.

The glued connections between the pieces may be hardened by UV light. It may then be the case that the lid 5 is glued to the wall 6 and hardened by UV light with the lid 5 under the wall 6, i.e. with the single-use bioreactor 1 being turned upside down. That limits the exposure of the light-sensitive optodes to the UV light if the UV light is applied from above.

The stirrer shaft 10 and the first impeller 62 and the second impeller 64 may be injection molded as well and may be separate pieces prior to the assembly. The stirrer shaft 10 may be extruded. Notably, the first rigid support structure 13 and/or the second rigid support structure 24 which may be extruded and post machined may be made of Eastar^{™} Copolyester MN211 or a similar material. Experiments have shown that extruding a support structure with conduits in these dimensions from a medical grade material is not trivial and other tested materials showed a stick-slip effect. A material staying within +- 10 % of one or more of the following properties is therefore preferred. This type of material is usually used for molding.
Specific Gravity according to ASTM D 792: 1.27
Water Absorption, 24 h immersion according to ASTM D 570 0.13 %
Tensile Stress @ Break according to ASTM D 638: 28 MPa (4100 psi)
Tensile Stress @ Yield according to ASTM D 638: 50 MPa (7300 psi)
Elongation @ Break according to ASTM D 638: 110 %
Elongation @ Yield according to ASTM D 638: 4.3 %
Flexural Strength according to ASTM D 790: 70 MPa (10200 psi)
Flexural Modulus according to ASTM D 790: 2100 MPa (300000 psi)
Rockwell Hardness, R Scale according to ASTM D 785: 106
Izod Impact Strength, Notched: @ 23°C (73°F) according to ASTM D 256: 101 J/m (1.9 ft.lbf/in.), @ -40°C (-40°F) according to ASTM D 256: 37 J/m (0.7 ft·lbf/in.)
Haze according to ASTM D 1003: 0.3 %
Regular Transmittance according to ASTM D 1003: 88 %
Total Transmittance according to ASTM D 1003: 91 %
Deflection Temperature: @ 0.455 MPa (66 psi) according to ASTM D 648: 70 °C (158 °F), @ 1.82 MPa (264 psi) according to ASTM D 648: 63 °C (145 °F)
Vicat Softening Temperature according to ASTM D 1525: 85 °C (185 °F)
Thermal Conductivity 0.19 W/m.K (1.3 Btu·in./h.ft2.°F)
Specific Heat: @ 240°C (464°F) according to ASTM D 2766: 2.01 kJ/kg K (0.48 Btu/lb·°F), @ 60°C (140°F) according to ASTM D 2766: 1.3 kJ/kg K (0.31 Btu/lb°F).

## Claims

1. Single-use closed bioreactor for culturing, fermenting or processing a biomass with a rigid housing (3) with an interior space (4) with an inner height, an inner width and a volume smaller 100 L, wherein the rigid housing (3) comprises a lid (5) with an upper surface (8) and a lower surface (9) and a body, wherein the body comprises a wall (6), a top and a closed bottom (7) opposite to the top, wherein the top of the body is irreversibly connected to the lid (5) such that the top is closed by the lid (5), wherein the lid (5) comprises a cooling structure (40) for cooling an exhaust gas of the bioreactor (1), wherein the cooling structure (40) comprises a cooling channel (41) with a channel ceiling (42) and a channel floor (43), wherein the cooling channel (41) has a channel inlet (44) at the lower surface (9) of the lid (5) within the interior space (4) of the housing and a channel outlet (45) at the upper surface (8) of the lid (5) outside the housing, wherein said cooling channel (41) has a horizontal section (46) which is at least partially oriented horizontally within the lid (5).

2. Single-use closed bioreactor according to claim 1, **characterized in that** the lid (5) consists of multiple parts with at least a top lid part (49) comprising the channel ceiling (42) and with a bottom lid part (50) comprising the channel floor (43), and that the bottom lid part (50) is attached to the top lid part (49) so that the cooling channel (41) is formed.

3. Single-use closed bioreactor according to claim 1 or 2, **characterized in that** the channel ceiling (42) and/or the channel floor (43) has an angled region (54) which is angled to a plane being perpendicular to the direction of gravity, such that a condensate flows towards the channel inlet (44).

4. Single-use closed bioreactor according to one of the preceding claims, **characterized in that** the channel inlet (44) has a distance to the wall (6) of at least 10 %, preferably at least 15 %, more preferably at least 20 % of a maximum width of the housing, and/or, **in that** the horizontal section (46) comprises at least one turn, in particular at least two turns.

5. Single-use closed bioreactor according to one of the preceding claims, **characterized in that**
the horizontal section (46) is located in a circular sector (55) of the lid (5) with an angle β smaller than 180°, preferably smaller than 150°, more preferably smaller than 120°, more preferably smaller than 100°, around the center axis of the stirrer shaft (10).

6. Single-use closed bioreactor according to one of the preceding claims, **characterized in that** the bottom through port (11) is located in the wall (6) of the body, preferably that the bottom through port (11) is located in the wall (6) of the body at a height smaller 25 %, preferably smaller than 15 %, more preferably smaller than 10 % of a maximum inner height.

7. Single-use closed bioreactor according to one of the preceding claims, **characterized in that** the bottom through port (11) comprises a bottom through port sensor (56) for determination of one or more parameters of the bioreactor (1), preferably, that the bottom through port sensor (56) is irreversibly connected to the bottom through port (11).

8. Single-use closed bioreactor according to one of the preceding claims, **characterized in that** the bottom through port (11) comprises a bottom through port channel (57) via which a fluid can be exchanged with the interior space (4) of the rigid housing (3), preferably, that the bottom through port channel (57) extends from the bottom through port (11) to the closed bottom (7) of the body, preferably, that the body comprises two bottom through ports (11), one with the bottom through port sensor (56) and one with the bottom through port channel (57).

9. Single-use closed bioreactor according to one of the preceding claims, **characterized in that** the lid (5) comprises a lid sensor port (60) configured to receive a sensor for determination of one or more parameters of the bioreactor (1).

10. Single-use closed bioreactor according to one of the preceding claims, **characterized in that** the closed bottom (7) comprises a bottom sensor port (61) configured to receive a bottom sensor for measuring one or more parameter of the bioreactor (1), preferably, the bottom sensor port (61) contains one or more optodes within the interior space (4) of the rigid housing (3) which are configured to interact non-invasively through the bottom sensor port (61) with the bottom sensor being located outside the body in the region of the bottom sensor port (61).

11. Single-use closed bioreactor according to one of the preceding claims, **characterized in that** different functionalities of the lid (5) are assigned to different sections of the lid (5), preferably, that the first inlet port (15) is assigned to a first lid section (65) and/or the second inlet port (26) is assigned to a second lid section (66), and/or the exhaust port (33) is assigned to a third lid section (67), and/or the lid sensor port (60) is assigned to a fourth lid section (68), and, that at least two of the first lid section (65) and the second lid section (66) and the third lid section (67) and the fourth lid section (68) do not overlap.

12. Single-use closed bioreactor according to one of the preceding claims, **characterized in that** the upper surface (8) of the lid (5) comprises stabilization ribs (69) for stabilizing the lid (5) which extend outwards from the upper surface (8) and/or that the lower surface (9) of the lid (5) comprises stabilization ribs (69) for stabilizing the lid (5) which extend in the interior space (4) of the rigid housing (3), preferably, that the lid (5) upper surface (8) and/or the lid (5) lower surface (9) comprises a first set of stabilization ribs (69) extending continuous or discontinuous radially from the center of the lid (5) and a second set of stabilization ribs (69) which interconnect the first set of stabilization ribs (69) with each other.

13. Bioreactor arrangement comprising a single-use bioreactor (1) according to one of the preceding claims, in particular pre-sterilized and arranged in a sterile packaging, preferably, wherein the bioreactor arrangement (2) comprises the second filter element (36) connectable to the sterile connector of the second Y-connector port (39), in particular packaged together with the single-use bioreactor (1).

14. Use of a single-use bioreactor (1) according to one of claims 1 to 12, preferably, wherein the single-use bioreactor (1) is in a use-state and connected to a cooling element, and/or, wherein the single-use bioreactor (1) is used for cell and gene therapy or perfusion or mammalian cell culture.

15. Method of production of a single-use bioreactor (1) according to one of claims 1 to 12, preferably, wherein the first rigid support structure (13) and/or the second rigid support structure (24) is extruded, and/or, wherein the wall (6) together with the closed bottom (7) and/or the top lid part (49) and/or the bottom lid part (50) are injection molded and preferably glued together.
